# EUROPEAN PATENT APPLICATION

(11) **EP 4 628 067 A1**
(43) Date of publication of application: **08.10.2025**
(21) Application number: 23897544.5
(22) Date of filing: 17.11.2023
(51) Int. Cl.: A61K 8/73, A61K 8/34, A61K 8/55, A61Q 5/12, A61Q 19/00

(54) **COMPOSITION**

(30) Priority: 01.12.2022 JP 2022192889
(71) Applicant: Shiseido Company, Ltd., Chuo-ku Tokyo 104-0061 (JP)
(72) Inventor: OKA, Takashi, Tokyo 104-0061 (JP); YOSHIMURA, Mika, Tokyo 104-0061 (JP); OKISHIMA, Anna, Tokyo 104-0061 (JP)
(74) Representative: Ter Meer Steinmeister & Partner
(86) International application number: PCT/JP2023/041420
(87) International publication number: WO 2024/116893

(57) **Abstract**

To provide a composition capable of increasing penetrability of hyaluronic acid into the skin, and of improving properties such as the flexibility of the stratum corneum, without requirement of complicated steps such as loading a hyaluronic acid component on a loading material.

A composition of the present disclosure comprises hydrogenated lecithin, hyaluronic acid, and a dihydric alcohol, wherein the mass ratio of the hyaluronic acid to the hydrogenated lecithin is more than 9.0 and less than 90.

## Description

### FIELD

The present disclosure relates to a composition containing hyaluronic acid and the like.

### BACKGROUND

For the purpose of, for example, moisturization of the skin, hyaluronic acid, which has a moisturizing function, has been utilized in the fields of cosmetics and the like.

PTL 1 describes a cosmetic comprising a hyaluronic acid- loaded nanoparticle in which hyaluronic acid is loaded in/on at least one of the insides or the surface of a nanoparticle formed of polylactic acid, polyglycolic acid, or a lactic acid-glycolic acid copolymer.

PTL 2 describes a skin external preparation, comprising a composite nanoparticle containing: (A) hyaluronic acid; and (B) a zwitterionic compound; wherein the particle diameter is 100 nm or less.

### [CITATION LIST]

### [PATENT LITERATURE]

[PTL 1] Japanese Unexamined Patent Publication (Kokai) No. 2010-150151
[PTL 2] WO 2018/182003

### SUMMARY

### [TECHNICAL PROBLEM]

For example, the stratum corneum, located in the outermost layer of the skin, is capable of maintaining its flexibility, and maintaining favorable skin conditions, as long as the moisture content in the stratum corneum is maintained. On the other hand, the stratum corneum under the favorable skin conditions has a barrier function that prevents entrance of foreign substances from the outside, and hence has a property that hardly allows penetration of effective components into the skin even when the components are applied to the skin. Therefore, simple application of a hyaluronic acid component to the skin merely causes retention of the hyaluronic acid component on the skin surface. As a result, in some cases, the application of a hyaluronic acid component to the skin does not lead to maintenance of a sufficient moisture content in the stratum corneum, resulting in decreased flexibility of the stratum corneum and failure to maintain favorable skin conditions.

The techniques described in PTLs 1 and 2 inevitably require preparation of hyaluronic acid into nanoparticles by loading the hyaluronic acid on a loading material such as polylactic acid, or by conjugating the hyaluronic acid with another material (polyion complexation). In both cases, preparation of a cosmetic requires complicated steps such as ultracentrifugation.

Accordingly, a main object of the present disclosure is to provide a composition capable of increasing penetrability of hyaluronic acid into the skin, and of improving properties such as the flexibility of the stratum corneum, without requirement of complicated steps such as loading a hyaluronic acid component on a loading material.

### [SOLUTION TO PROBLEM]

### <Aspect 1>

A composition comprising:
hydrogenated lecithin;
hyaluronic acid; and
a dihydric alcohol,
wherein the mass ratio of the hyaluronic acid to the hydrogenated lecithin is more than 9.0 and less than 90.

### <Aspect 2>

The composition according to aspect 1, wherein the content of phosphatidylcholine among constituent lipids of the hydrogenated lecithin is 40% or more.

### <Aspect 3>

The composition according to aspect 1 or 2, wherein the content of the hyaluronic acid is 1.0% by mass or less with respect to the total amount of the composition.

### <Aspect 4>

The composition according to any one of aspects 1 to 3, wherein the content of the hydrogenated lecithin is 0.001% by mass or more with respect to the total amount of the composition.

### <Aspect 5>

The composition according to any one of aspects 1 to 4, wherein the content of the dihydric alcohol is 0.01% by mass or more with respect to the total amount of the composition.

### <Aspect 6>

The composition according to any one of aspects 1 to 5, wherein the dihydric alcohol is at least one selected from the group consisting of dipropylene glycol and 1,3-butylene glycol.

### <Aspect 7>

The composition according to any one of aspects 1 to 6, for use in penetration of the hyaluronic acid into body surface or body hair.

### <Aspect 8>

A cosmetic comprising the composition according to any one of aspects 1 to 7.

### [ADVANTAGEOUS EFFECTS OF INVENTION]

The present disclosure can provide a composition capable of increasing the penetrability of hyaluronic acid into the skin, and improving properties such as the flexibility of the stratum corneum, without requirement of complicated steps such as loading a hyaluronic acid component on a loading material.

### BRIEF DESCRIPTION OF DRAWINGS

Fig. 1 is a graph illustrating changes in the elastic modulus of the stratum corneum in cases where, for example, a composition containing hydrogenated lecithin, hyaluronic acid, and a dihydric alcohol (1,3-butylene glycol) was used.
Fig. 2 is a graph illustrating changes in the elastic modulus of the stratum corneum in cases where, for example, a composition containing hydrogenated lecithin, hyaluronic acid, and a trihydric alcohol (glycerin) was used.
Fig. 3 is a graph illustrating changes in the elastic modulus of the stratum corneum in systems using hyaluronic acid alone or hydrogenated lecithin alone, or in a system using a mixture of hyaluronic acid and hydrogenated lecithin.
Fig. 4 is a graph illustrating changes in the elastic modulus of the stratum corneum in cases using the same content of hyaluronic acid, but using different mass ratios of the hyaluronic acid to hydrogenated lecithin.
Fig. 5 is a graph illustrating a change in the elastic modulus of the stratum corneum in cases using the same content of hydrogenated lecithin, but using different mass ratios of hyaluronic acid to the hydrogenated lecithin.
Fig. 6 is a graph illustrating changes in the hyaluronic acid concentration in the two layers or four layers from the surface of the stratum corneum in a case where a composition of one embodiment of the present disclosure was applied to the skin, the composition having a mass ratio of hyaluronic acid to hydrogenated lecithin of 30.
Fig. 7 is a graph illustrating the total amount of hyaluronic acid contained in the stratum corneum in a case where a composition of one embodiment of the present disclosure was applied to the skin, the composition having a mass ratio of hyaluronic acid to hydrogenated lecithin of 30.
Fig. 8 is a graph illustrating changes in the hyaluronic acid concentration in the two layers or four layers from the surface of the stratum corneum in cases where a composition was applied to the skin, the composition having a mass ratio of hyaluronic acid to hydrogenated lecithin outside the scope of the present disclosure.
Fig. 9 is a graph illustrating the total amount of hyaluronic acid contained in the stratum corneum in cases where a composition was applied to the skin, the composition having a mass ratio of hyaluronic acid to hydrogenated lecithin outside the scope of the present disclosure.

### DESCRIPTION OF EMBODIMENTS

Hereinafter, embodiments of the present disclosure are described in detail. The present disclosure is not limited to the following embodiments, and may be variously modified and practiced within the scope of the present invention.

The composition of the present disclosure comprises hydrogenated lecithin, hyaluronic acid, and a dihydric alcohol, and the mass ratio of the hyaluronic acid to the hydrogenated lecithin is preferably more than 9.0 and less than 90.

Although the present invention is not limited by any principle, in cases where the composition of the present disclosure comprises the dihydric alcohol, and comprises the hyaluronic acid and the hydrogenated lecithin at the prescribed ratio, the following action principle is thought to enable the improvement of the penetrability of hyaluronic acid into the skin, and the improvement of properties of the stratum corneum such as the flexibility.

Since amphoteric surfactants have an action to temporarily destabilize the orderliness of intercellular lipids in the stratum corneum of the skin, the present inventors thought that use of lecithin, which is a natural amphoteric surfactant, may promote penetration of hyaluronic acid into the skin.

Hyaluronic acid is soluble in water, but lecithin is hardly soluble in water. Therefore, the present inventors first performed a study on solvents in which lecithin is soluble, to be used for mixing hyaluronic acid with lecithin. As a result, the present inventors discovered that 1,3-butylene glycol and glycerin can be used as solvents that allow the mixing of hyaluronic acid with hydrogenated lecithin. However, in a system using glycerin, mixing of hyaluronic acid with hydrogenated lecithin did not improve the penetrability of the hyaluronic acid into the skin and properties of the stratum corneum such as the flexibility. On the other hand, the present inventors discovered that, in cases where 1,3-butylene glycol is used as the solvent, and hyaluronic acid and hydrogenated lecithin are used at a prescribed ratio, the penetrability of the hyaluronic acid into the skin and properties of the stratum corneum such as the flexibility, can be improved even without carrying out complicated processes such as those described in PTLs 1 and 2, including ultracentrifugation. This is thought to be due to the following reason.

While glycerin is a trihydric alcohol, 1,3-butylene glycol is a dihydric alcohol, which has one less hydroxyl group than glycerin. Dihydric alcohols are thought to have weaker hydrophilic properties than trihydric alcohols. Therefore, hydrogenated lecithin is thought to exhibit different behaviors in a solvent depending on whether the solvent is a dihydric alcohol or trihydric alcohol, and such a difference is thought to affect the penetrability of the hyaluronic acid into the skin and properties of the stratum corneum such as the flexibility.

More specifically, in compositions, hyaluronic acid is typically present in the form of a spread thread. However, in case where a dihydric alcohol such as 1,3-butylene glycol is used as the solvent of the hydrogenated lecithin, and the hydrogenated lecithin and the hyaluronic acid are used at the prescribed ratio, the hyaluronic acid may interact with the hydrogenated lecithin to have, for example, a form in which the hyaluronic acid easily penetrates into the skin (such as a form in which the hyaluronic acid has become small due to suppression of its spreading), or in addition, the hydrogenated lecithin may act, for example, to temporarily destabilize the orderliness of intercellular lipids in the skin, resulting in the improvement of the penetrability of the hyaluronic acid into the skin and properties of the stratum corneum such as the flexibility.

### <<Composition>>

The composition of the present disclosure comprises hydrogenated lecithin, hyaluronic acid, and a dihydric alcohol. Since the composition of the present disclosure uses the dihydric alcohol, and comprises the hydrogenated lecithin and the hyaluronic acid at the prescribed mixing ratio, properties such as the penetrability of hyaluronic acid into the skin and the flexibility of the stratum corneum can be improved.

### <Hydrogenated Lecithin>

Among phospholipids, lecithin is a type of glycerophospholipid, and is known as a major component of the biological membranes. Known lecithins that are industrially available include soybean lecithin, which is derived from soybean, and egg yolk lecithin, which is derived from egg yolk, and the like. The lecithin used in the composition of the present disclosure is lecithin that has been subjected to hydrogenation, that is, hydrogenated lecithin. In particular, the hydrogenated lecithin is preferably a hydrogenation product of soybean lecithin, derived from soybean, from the viewpoint of the penetrability of the hyaluronic acid into the skin, improvement of the flexibility of the stratum corneum, and the like. The later-described dihydric alcohol favorably enables mixing of the hydrogenated lecithin with the hyaluronic acid. A single type of hydrogenated lecithin may be used, or a combination of two or more types of hydrogenated lecithins may be used.

From the viewpoint of the penetrability of the hyaluronic acid into the skin, and improvement of the flexibility of the stratum corneum, and the like, the content of phosphatidylcholine among the constituent lipids of the hydrogenated lecithin is preferably 40% or more, more preferably 50% or more, 60% or more, 70% or more, or 75% or more. There is no upper limit of the content of phosphatidylcholine, and the content may be, for example, 100% or less, less than 100%, 95% or less, 90% or less, or 85% or less. The content of phosphatidylcholine in the hydrogenated lecithin can be determined, for example, by the thinlayer chromatography (TLC) method, the ³¹P-NMR method, or a method in which hydrogen peroxide generated using phospholipase is quantified by coloration or fluorescence. Among these, the method in which hydrogen peroxide generated using phospholipase is quantified by coloration or fluorescence is most preferred.

Further, from the viewpoint of compatibility with the dihydric alcohol, the hydrogenated lecithin preferably has an iodine number of 10 or less. The "iodine number" herein is the number of grams of iodine bound to 100 g of the phospholipid (hydrogenated lecithin), and can be measured according to the method described in the "Iodine Number" section of the Testing Method for Fats and Oils in the Japanese Pharmacopoeia.

The content of the hydrogenated lecithin may be, for example, 0.0001% by mass or more, 0.0005% by mass or more, 0.001% by mass or more, 0.002% by mass or more, 0.003% by mass or more, 0.004% by mass or more, or 0.005% by mass or more, and may be, for example, 5.0% by mass or less, 3.0% by mass or less, 1.0% by mass or less, 0.50% by mass or less, 0.10% or less, 0.07% by mass or less, or 0.05% by mass or less, with respect to the total amount of the composition. In particular, from the viewpoint of the penetrability of the hyaluronic acid into the skin, improvement of the flexibility of the stratum corneum, and the like, the content of the hydrogenated lecithin is preferably 0.002% by mass or more, more preferably 0.004% by mass or more, or 0.005% by mass or more, and is preferably 0.05% by mass or less, more preferably 0.03% by mass or less, 0.02% by mass or less, or 0.01% by mass or less.

### <Hyaluronic Acid>

The hyaluronic acid of the present disclosure is not limited. In general, hyaluronic acid means a linear polymer in which N-acetyl-D-glucosamine residues and D-glucuronic acid residues are alternately linked, and such hyaluronic acid can be obtained, for example, by isolation extraction from a cockscomb or another animal tissue, or by fermentation using a microorganism such as the genus *Streptococcus.*

The hyaluronic acid may be a derivative of hyaluronic acid. Examples of the hyaluronic acid derivative that may be used include hyaluronic acid salts, more specifically, metal salts of hyaluronic acid, such as the sodium salt of hyaluronic acid, the potassium salt of hyaluronic acid, the magnesium salt of hyaluronic acid, the calcium salt of hyaluronic acid, and the aluminum salt of hyaluronic acid; and also include derivatives of hyaluronic acid and hyaluronic acid salts obtained by etherification, esterification, amidation, acetylation, acetalization, or ketalization of hydroxyl, carboxyl, or the like of hyaluronic acid. Here, "hyaluronic acid" in the present disclosure may encompass the concept of hyaluronic acid and derivatives thereof.

The weight average molecular weight of the hyaluronic acid is not limited, and may be, for example, 10,000,000 or less. Not only hyaluronic acid, but also other low-molecular-weight drugs are generally considered to easily penetrate into the skin. Since a low-molecular-weight hyaluronic acid hardly stays in the skin, and its water-retaining performance is lower than that of a high-molecular-weight hyaluronic acid, it may be difficult for the low-molecular-weight hyaluronic acid to maintain the moisture-retaining effect in the skin over a long period of time. Therefore, for the purpose of maintaining the moisture-retaining effect in the skin over a long period of time, it is advantageous to use a high-molecular-weight hyaluronic acid. From the viewpoint of the penetrability of the hyaluronic acid into the skin, the water-retaining performance of the hyaluronic acid, improvement of the flexibility of the stratum corneum, and the like, the weight average molecular weight of the hyaluronic acid may be, for example, 500 or more, 1000 or more, 5000 or more, 10,000 or more, 50,000 or more, 100,000 or more, 300,000 or more, 500,000 or more, 800,000 or more, or 1,000,000 or more, and may be, for example, 10,000,000 or less, 8,000,000 or less, 5,000,000 or less, 3,000,000 or less, 2,000,000 or less, or 1,500,000 or less. Here, the weight average molecular weight means the weight average molecular weight in terms of polystyrene in gel permeation chromatography measurement.

As the hyaluronic acid, hyaluronic acid and its derivatives may be used individually, or as a combination of two or more thereof. The hyaluronic acid and/or its derivative(s) used may have either the same molecular weight or different molecular weights.

A commercially available hyaluronic acid may be used. Examples of the commercially available hyaluronic acid include hyaluronic acid HA-LQ (manufactured by Kewpie Corporation), hyaluronic acid FCH (manufactured by Kikkoman Biochemical Company), hyaluronic acid IW120 (manufactured by Iwaki & Co., Ltd.), and sodium bio-hyaluronate HA12N (manufactured by Shiseido Co., Ltd.).

The content of the hyaluronic acid may be, for example, 0.005% by mass or more, 0.01% by mass or more, 0.05% by mass or more, 0.09% by mass or more, more than 0.09% by mass, 0.10% by mass or more, 0.15% by mass or more, 0.20% by mass or more, 0.25% by mass or more, 0.30% by mass or more, 0.35% by mass or more, 0.40% by mass or more, or 0.45% by mass or more, and may be, for example, 5.0% or less, 4.0% or less, 3.0% or less, 2.0% or less, 1.0% by mass or less, 0.90% by mass or less, 0.80% by mass or less, 0.70% by mass or less, 0.60% by mass or less, 0.50% by mass or less, 0.45% by mass or less, less than 0.45%, 0.40% by mass or less, or 0.35% by mass or less, with respect to the total amount of the composition. In particular, from the viewpoint of the penetrability of the hyaluronic acid into the skin, improvement of the flexibility of the stratum corneum, and the like, the content of the hyaluronic acid is preferably 1.0% by mass or less, more preferably 0.45% by mass or less, or less than 0.45%, and is preferably 0.09% by mass or more, or more than 0.09% by mass, more preferably 0.10% by mass or more.

### <Dihydric Alcohol>

Examples of the dihydric alcohol include aliphatic or alicyclic compounds in which the two hydroxy groups are bound to two different carbon atoms. A single dihydric alcohol may be used, or a combination of two or more dihydric alcohols may be used.

Specific examples of the dihydric alcohol include at least one type of glycol component selected from the group consisting of ethylene glycol, propylene glycol, trimethylene glycol, 1,2-butylene glycol, 1,3-butylene glycol, tetramethylene glycol, 2,3-butylene glycol, pentamethylene glycol, hexylene glycol, octylene glycol, dipropylene glycol, and polyethylene glycol. In particular, from the viewpoint of the penetrability of the hyaluronic acid into the skin, improvement of the flexibility of the stratum corneum, and the like, dipropylene glycol and 1,3-butylene glycol are preferred, and 1,3-butylene glycol is more preferred.

The content of the dihydric alcohol may be, for example, 0.01% by mass or more, 0.05% by mass or more, 0.1% by mass or more, 0.3% by mass or more, 0.5% by mass or more, 1.0% by mass or more, 3.0% by mass or more, or 5.0% by mass or more, with respect to the total amount of the composition. There is no upper limit of the content, and the content may be, for example, 50% by mass or less, 40% by mass or less, 30% by mass or less, 20% by mass or less, 15% by mass or less, or 10% by mass or less. In particular, from the viewpoint of the penetrability of the hyaluronic acid into the skin, improvement of the flexibility of the stratum corneum, and the like, the content of the dihydric alcohol is preferably 1.0% by mass or more, more preferably 5.0% by mass or more, and is preferably 30% by mass or less, more preferably 20% by mass or less, 15% by mass or less, or 10% by mass or less.

### <Mass Ratio of Hyaluronic Acid to Hydrogenated Lecithin>

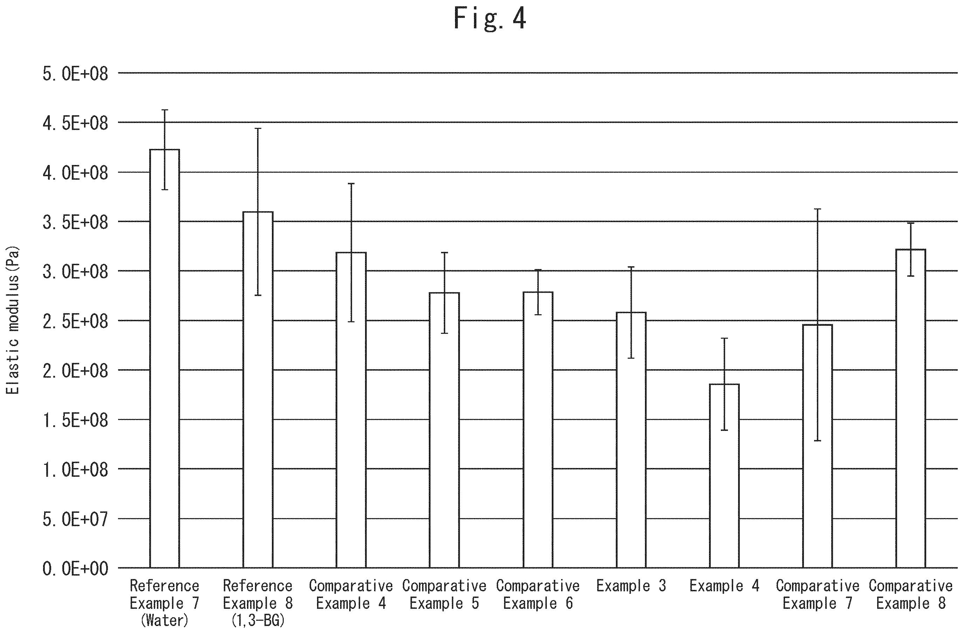

The composition of the present disclosure comprises hydrogenated lecithin, hyaluronic acid, and a dihydric alcohol, and the mass ratio of the hyaluronic acid to the hydrogenated lecithin is preferably more than 9.0 and less than 90. The upper limit of the mass ratio was tentatively set based on the result that the composition of Comparative Example 7 in Fig. 4 showed no statistically significant difference according to the t-test. However, according to the error bar for Comparative Example 7 in Fig. 4, the composition of Comparative Example 7 can potentially be an Example depending on the sample number or the like. Therefore, regarding the upper limit of the mass ratio of the hyaluronic acid to the hydrogenated lecithin in the composition of the present disclosure, the mass ratio can potentially be set to less than 270, 250 or less, 200 or less, 150 or less, 100 or less, or 90 or less.

From the viewpoint of the penetrability of the hyaluronic acid into the skin, improvement of the flexibility of the stratum corneum, and the like, the mass ratio of the hyaluronic acid to the hydrogenated lecithin in the composition of the present disclosure is preferably more than 9.0, 9.5 or more, or 10 or more, more preferably 12 or more, 15 or more, or 17 or more, and is preferably less than 90, 85 or less, 80 or less, or 75 or less, more preferably 70 or less, 65 or less, or 60 or less.

### <Aqueous Medium>

The composition of the present disclosure may typically contain an aqueous medium. The aqueous medium is not limited, and aqueous media used in cosmetics, quasi-drugs, and the like may be used. Examples of the aqueous media that may be used include ion-exchanged water, distilled water, ultrapure water, tap water, and buffers. These aqueous media may be used individually, or as a combination of two or more thereof.

Examples of the buffers include citrate buffer, lactate buffer, phosphate buffer, acetate buffer, tartrate buffer, borate buffer, and Tris buffer. From the viewpoint of securing a high buffer capacity, citrate buffer, lactate buffer, and phosphate buffer are preferred. Citrate buffer is more preferred.

The pH of the buffer may be, for example, 9.0 or less, 8.5 or less, 8.0 or less, 7.5 or less, 7.0 or less, 6.8 or less, or 6.5 or less. There is no lower limit of the pH of the buffer. From the viewpoint of, for example, reducing irritation to the skin, the pH is preferably 4.5 or more, 5.5 or more, or 6.0 or more.

### <Optional Components>

In the composition of the present disclosure, various components may be included, when appropriate, as long as the effect of the present invention is not adversely affected. Examples of such components include salts; humectants; surfactants (anionic surfactants, cationic surfactants, amphoteric surfactants, and nonionic surfactants); skin or hair nutrients; vitamins; water-soluble agents applicable to pharmaceuticals, quasi-drugs, cosmetics, and the like; ultraviolet absorbers; antioxidants; preservatives; antioxidant aids; thickeners; pigments; dyes; colorants; and fragrances. These optional components may be used individually, or as a combination of two or more thereof.

### (Salt)

The composition of the present disclosure may contain a salt. A single salt may be used, or a combination of two or more salts may be used. The "salt" in the present disclosure does not encompass hyaluronic acid salts.

The salt that may be included in the composition is not limited, and examples of the salt include at least one salt selected from the group consisting of inorganic salts and organic acid salts. For example, taking the use as a cosmetic into account, the salt, among inorganic salts and organic acid salts, is preferably a salt that hardly adversely affects the skin. Here, the term "inorganic salt" means a salt comprising only inorganic components, and this can also be said to be a salt formed by ions generated from an inorganic acid and an inorganic base. The term "organic acid salt" means a salt formed by an organic acid and a metal ion bound to each other. The salt in the composition is generally present in the form of ions derived from the salt. Thus, in the present disclosure, the term "composition containing a salt" means, for example, that the salt is contained in the form of such ions. Ionic surfactants are not included in the "salt" in the present disclosure.

Examples of the salt that may be used include at least one selected from the group consisting of monovalent salts, divalent salts, and trivalent salts.

The inorganic salts are preferably at least one selected from the group consisting of sodium salts, potassium salts, calcium salts, magnesium salts, and aluminum salts, more preferably at least one selected from the group consisting of sodium salts and magnesium salts.

Specific examples of the inorganic salts include sodium nitrate, sodium sulfate, sodium chloride, potassium nitrate, potassium sulfate, potassium chloride, calcium nitrate, calcium sulfate, calcium chloride, magnesium nitrate, magnesium sulfate, magnesium chloride, aluminum nitrate, aluminum sulfate, and aluminum chloride. Sodium chloride and magnesium chloride are especially preferred.

Specific examples of the organic acid salts include citrate, acetate, lactate, tartrate, succinate, malate, glycolate, salicylate, and pyrrolidone carboxylate. Specific examples thereof include salts in which an organic acid such as citric acid, acetic acid, lactic acid, tartaric acid, succinic acid, malic acid, glycolic acid, salicylic acid, or pyrrolidone carboxylic acid is bound to a metal ion such as sodium ion, potassium ion, calcium ion, magnesium ion, or aluminum ion.

The ionic strength of the salt in the composition may be, for example, 0.05 or more, 0.06 or more, 0.07 or more, or 0.08 or more, and may be 5.0 or less, 4.0 or less, 3.0 or less, 2.0 or less, 1.0 or less, 0.7 or less, 0.5 or less, 0.4 or less, or 0.3 or less.

Here, for example, when the composition is prepared by adding a salt to a buffer, the ionic strength of the salt is calculated based on all salt components, including the salt component (s) contained in the buffer itself and the salt component(s) separately added to the buffer.

### (Humectant)

The composition of the present disclosure may contain a humectant. A single humectant may be used, or a combination or two or more humectants may be used. The term "humectant" herein does not encompass hyaluronic acid and dihydric alcohols.

Examples of the humectant include PEG/PPG-17/4 dimethyl ether.

The amount of the humectant included in the composition may be, for example, 1.0% by mass or more, 3.0% by mass or more, 5.0% by mass or more, or 7.0% by mass or more, and may be 30% by mass or less, 25% by mass or less, 20% by mass or less, or 15% by mass or less, with respect to the total amount of the composition.

### <<Method of Producing Composition>>

An example of the method of producing the composition of the present disclosure is described below, but the method of producing the composition is not limited thereto. The various components described above may be also used in the method of producing the composition.

Hyaluronic acid is mixed with water to prepare an aqueous hyaluronic acid solution. Further, a dihydric alcohol is mixed with hydrogenated lecithin to prepare a hydrogenated lecithin solution. Subsequently, the aqueous hyaluronic acid solution is mixed with the hydrogenated lecithin solution. By this, the composition of the present disclosure can be produced. The preparation of the composition may be carried out, when necessary, under warming or cooling.

The mixing of the aqueous hyaluronic acid solution with the hydrogenated lecithin solution may be carried out by adding the hydrogenated lecithin solution to the aqueous hyaluronic acid solution, or may be carried out by adding the aqueous hyaluronic acid solution to the hydrogenated lecithin solution. From the viewpoint of the penetrability of the hyaluronic acid into the skin, improvement of the flexibility of the stratum corneum, and the like, the mixing is preferably carried out by adding the hydrogenated lecithin solution to the aqueous hyaluronic acid solution.

### <<Uses of Composition>>

Uses of the composition of the present disclosure are not limited, and examples of the uses include cosmetics (cosmetic products), pharmaceuticals, and quasi-drugs.

For example, a cosmetic containing the composition of the present disclosure may be provided in a form in which it is contained in various containers. The formulation of the cosmetic is not limited, and examples of the formulation include a liquid, an emulsion, a cream, a gel, a spray, and a mousse. These may be in the form of a single-phase system, or may be in the form of a two-phase system of a non-emulsified or emulsified, oil-in-water or water-in-oil type. The term "spray" in the present disclosure may encompass mist-type sprays, aerosol-type sprays, and the like.

The product form of the cosmetic is also not limited, and examples of the product form include: skin care cosmetics such as emulsions, creams, face oils, body oils, lotions, and beauty essences; make-up cosmetics such as foundations, make-up bases, lipsticks, blushers, eye shadows, mascaras, and mascara bases; sunscreen cosmetics; body cosmetics; aromatic cosmetics; skin-washing agents such as make-up removers, facial cleansers, and body shampoos; and hair cosmetics such as hair sprays, hair creams, hair lotions, hair conditioners, and shampoos. In particular, the cosmetic can be advantageously used as a leave-on type cosmetic that focuses on penetration of hyaluronic acid into the skin or the like, and that can be used without rinsing.

### <<Application Site of Composition>>

The composition of the present disclosure is applicable to any part of the body. For example, the cosmetic may be applied to any part on the skin surface (body surface) or on the body hair (hair). More specifically, the composition of the present disclosure may be applied, when appropriate, to the skin surface of the face (lips, eyes, eyelids, cheeks, forehead, glabella, nose, or the like), head (scalp), ears, hands, arms, neck, legs, feet, chest, abdomen, back, or the like, or to a body hair such as scalp hair, eyelashes, eyebrows, or beards. Here, the skin also includes nails and the like formed by hardening of the corneum of the skin epidermis. In the present disclosure, "body hair" has the same meaning as "hair", and encompasses all kinds of hair on the body. Specific examples of the body hair (hair) include scalp hair, eyelashes, eyebrows, and beards.

Thus, the composition of the present disclosure can be suitably used as a composition for the body surface or body hair. Further, since the composition of the present disclosure suitably allows penetration of hyaluronic acid into the body surface or body hair, the composition can be suitably used as a composition for penetration of hyaluronic acid into the body surface or body hair.

### <<Beauty Method Using Composition>>

The composition of the present disclosure can be used also as a cosmetic or the like as described above. The beauty method using the composition (for example, cosmetic) of the present disclosure comprises applying the composition to the body surface or body hair. In the present disclosure, "beauty method" means a method for achieving a beautiful and orderly condition of the body surface or body hair by application of a composition to the body surface or body hair. The method is thus different from a method of operation, treatment, or diagnosis of a human.

In general, one unknowingly suffers from deprivation of moisture from skin or body hair when the skin or body hair is exposed to dryness, which deprivation leads to a state where the moisture content on the surface of the skin or body hair cannot be maintained. For example, when the moisture on the skin surface is insufficient, a moisturizing component to be produced by the skin itself (Natural Moisturizing Factor (NMF)) cannot be successfully produced. As a result, the barrier function and the moisturizing function on the skin surface deteriorate, and the skin tends to be damaged. This has been thought to result in loss of moisture, formation of wrinkles, skin roughness, and the like. Further, it has been thought that dryness of hairs due to loss of moisture causes formation of split ends, hair breakage, easy spreading of the hairs due to static electricity, and the like.

Since, for example, the cosmetic comprising the composition of the present disclosure allows favorable penetration of hyaluronic acid into the skin or body hair, the moisture in the skin or body hair can be favorably retained by the cosmetic. As a result, for example, the function that allows the skin itself to generate the moisturizing component can be improved, and moreover, poor turnover in the stratum corneum can be improved, or moisture of hair can be improved. Therefore, troubles such as skin roughness, or formation of split ends or hair breakage can be reduced to enhance the beauty effect.

The means of application of the composition to the body surface or body hair is not limited. For example, the application may be performed by applying the composition onto the body surface or body hair. Regarding the means of applying the composition onto the body surface or body hair, the application may be carried out by spraying the composition onto the body surface or body hair using a spray container to which the cosmetic has been fed. Alternatively, the application may be carried out by feeding the composition to a container having no spray function, collecting an appropriate amount of the composition from the container onto a finger, palm, or the like, and then spreading the composition on the body surface or body hair.

From the viewpoint of enhancing the beauty effect, the application of the composition to the body surface or body hair is preferably carried out for 2 days or more, 5 days or more, 7 days or more, 10 days or more, 15 days or more, 20 days or more, or 30 days or more. From the viewpoint of even enhancing the beauty effect, the application of the composition to the body surface or body hair is more preferably carried out continuously for 2 days or more, 5 days or more, 7 days or more, 10 days or more, 15 days or more, 20 days or more, or 30 days or more. Here, the continuous application may be referred to as "continuous use".

### EXAMPLES

Test Examples and Examples are described below to illustrate the present invention in more detail. However, the present invention is not limited thereto. Unless otherwise specified, each content is hereinafter expressed in % by mass. The various evaluation methods described in the Examples are applicable not only to the compositions described in the Examples, but also to compositions containing the components described above.

### <<Test Examples 1 to 7>>

### <Evaluation of Compositions>

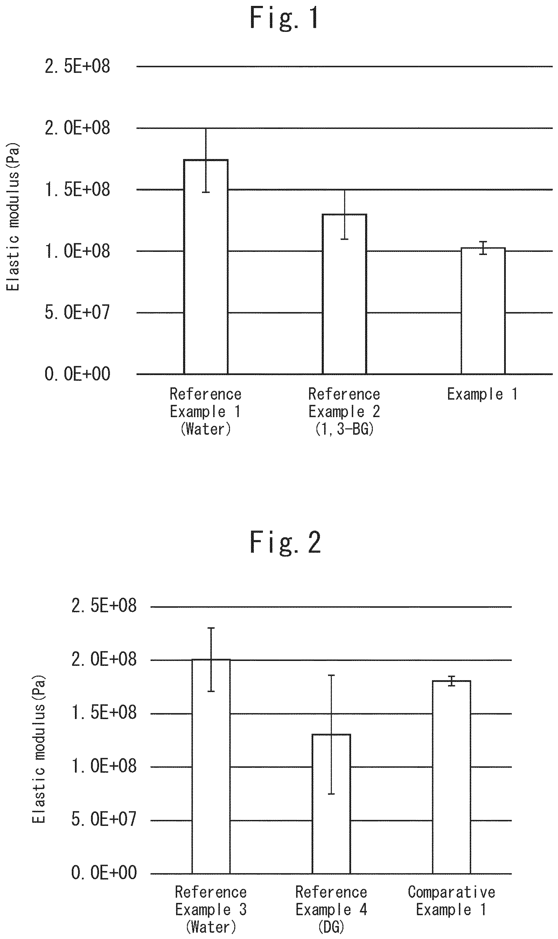

The evaluations described below were carried out using compositions obtained by the following production methods. The results are summarized in Tables 1 to 7 and Figs. 1 to 9. In the tables and figures, "HA" means hyaluronic acid; "BG" means butylene glycol; and "DG" means glycerin (dynamite glycerin). "Elastic modulus" means the dynamic storage modulus (E'), and "mass ratio" means the mass ratio of the hyaluronic acid to the hydrogenated lecithin.

### (Evaluation of Flexibility of Stratum Corneum: Elastic Modulus)

Measurement of the viscoelasticity of the stratum corneum was carried out using the dynamic viscoelasticity measurement apparatus DVA-220 (manufactured by IT Keisoku Seigyo Co., Ltd.), which is a horizontally driven rheometer. Both ends of a stratum corneum piece of human skin, to which a composition sample had been preliminarily applied, were held horizontally by two clamps placed in the measurement section of the apparatus. The measurement section has a temperature-humidity-controllable chamber structure, which was set to a temperature of 32°C, which corresponds to the skin surface temperature, and a relative humidity of 50%, which corresponds to a humidity during the dry season. With one clamp, a sinusoidal stress with a fixed amplitude (strain, 0.2%; 1 Hz) was applied to one end of the stratum corneum. The other clamp is equipped with a high sensitivity sensor that detects a weak stress transmitted through the stratum corneum. The detected stress signal is converted to the dynamic storage modulus (E') and the dynamic loss modulus (E"), of which the dynamic storage modulus (E') was used as an index of the softness of the stratum corneum. Thus, the smaller the value of the dynamic storage modulus (E'), the softer the stratum corneum has become.

The graphs of the elastic modulus in Figs. 1 to 5 were prepared by calculating the dynamic storage modulus (E') using three different stratum corneum pieces for each sample, and performing the t-test for the resulting values. Each bar shows the intermediate value (the mean of the three values) of each error bar. The grades "A" to "C" in each table are based on the result of the t-test of the values of the dynamic storage modulus (E') of each composition obtained, wherein the test was carried out using, as a control, the result for the composition containing the solvent for dissolving the hydrogenated lecithin, but not containing the hyaluronic acid and the hydrogenated lecithin. The grade "A" herein means a state where the bar shown in the figure is lower than that of the control, and where there is a statistically significant difference based on the t-test, indicating that the stratum corneum has increased flexibility. The grade "B" herein means that, although the bar shown in the figure is lower than that of the control, there is only a small statistically significant difference based on the t-test, indicating that the stratum corneum has weak flexibility, or that the stratum corneum is unlikely to have increased flexibility. The grade "C" herein means that the bar shown in the figure is not lower than that of the control, and/or that there is no statistically significant difference based on the t-test, indicating that the stratum corneum has no increased flexibility, or that the stratum corneum is very unlikely to have increased flexibility.

### (Evaluation of Penetrability of Hyaluronic Acid)

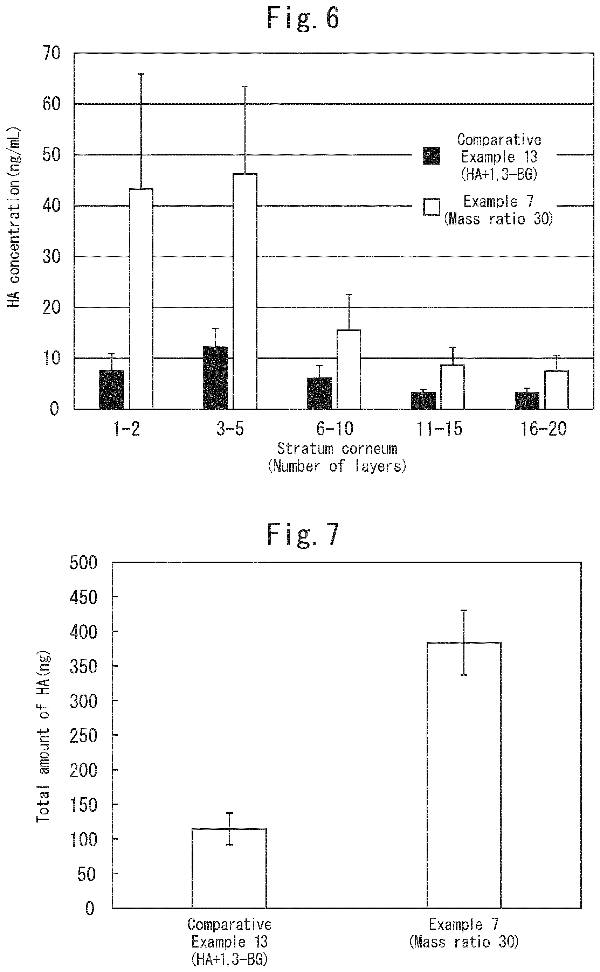

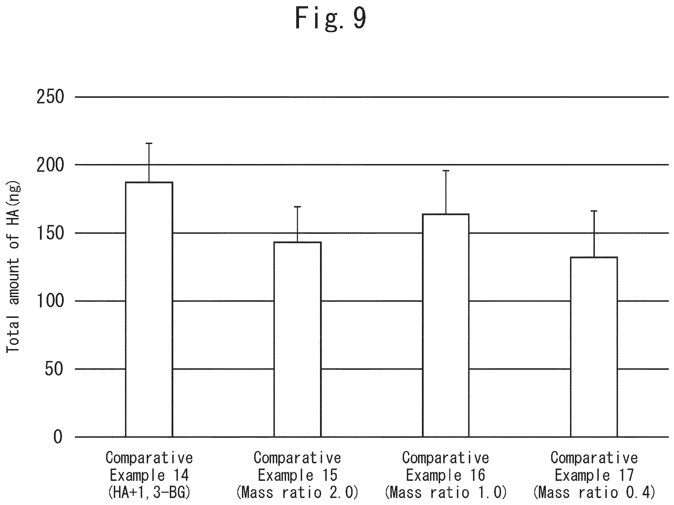

Excised human skin was placed between the donor section and the receptor section of a diffusion cell array system. The effective diffusion area of the excised human skin at this time was 0.785 cm². The receptor section was filled with 1.2 mL of a receptor solution (PBS solution), and the receptor solution was continuously stirred with a stirrer. While the surface of the excised human skin was kept at 32°C, 10 µL/cm² of a cosmetic was applied to the donor section. Twenty-four hours thereafter, the excised human skin was removed, and its surface was washed with an aqueous soap solution and purified water, followed by peeling off the stratum corneum 20 times using D-Squame (trademark) discs (Clinical & Derm LLC.). The discs after the peeling off were immersed in a 5% aqueous methanol solution, and sonication was performed for 20 minutes. Thereafter, hyaluronic acid was quantified by the ELISA method, and the amount of hyaluronic acid contained in the stratum corneum on two discs or four discs was calculated in each case. "Total amount of HA" in Fig. 7 and Fig. 9 is the total amount of hyaluronic acid contained in the stratum corneum on the 20 discs. Each value in the tables and figures is the average of the amount of hyaluronic acid obtained by three or four times of measurement.

Regarding "Good" and "Poor" for the item "Penetrability of HA" in Tables 6 and 7, each composition was rated as "Good" in cases where the total amount of HA was larger than that in the control containing no hydrogenated lecithin, that is, Comparative Examples 13 or 14 in Fig. 7 and Fig. 9, while each composition was rated as "Poor" in cases where the total amount of HA was smaller than that in the control. A composition rated as "good" can be said to have allowed favorable penetration of hyaluronic acid into the stratum corneum.

### <Test Example 1: Effect of Solvent 1>

In Test Example 1, a study was carried out on the effect of a dihydric alcohol (1,3-butylene glycol) as a solvent that dissolves the hydrogenated lecithin. The results are shown in Table 1 and Fig. 1.

### (Reference Example 1)

Ion-exchanged water was used as a test sample of Reference Example 1.

### (Reference Example 2: Control)

Ion-exchanged water was mixed with 1,3-butylene glycol to prepare a test sample of Reference Example 2.

### (Example 1)

Hyaluronic acid was mixed with water to prepare an aqueous hyaluronic acid solution, and 1,3-butylene glycol was mixed with hydrogenated lecithin to prepare a hydrogenated lecithin solution. Subsequently, under warming as appropriate, the hydrogenated lecithin solution was added to and mixed with the aqueous hyaluronic acid solution, to prepare a test sample of Example 1.

### [Table 1]

**Table 1**

| Component | Component name | Reference Example 1 | Reference Example 2 | Example 1 |
|---|---|---|---|---|
| Water | Ion-exchanged water | 100 | 90 | 89.72 |
| Dihydric alcohol | 1,3-Butylene glycol | | 10 | 10 |
| Hyaluronic acid | Hyaluronic acid | | | 0.27 |
| Lecithin | Hydrogenated lecithin ¹⁾ | | | 0.01 |
| Total | | 100 | 100 | 100 |
| Mass ratio | | - | - | 27 |
| Evaluation | Flexibility of stratum corneum | C | - | A |

| | | | | |
|---|---|---|---|---|
| 1) Content of phosphatidylcholine: 75 to 85%; iodine number: 10 or less | | | | |

### <Results>

From the results in Table 1 and Fig. 1, it could be confirmed that the dihydric alcohol (1,3-butylene glycol) can be used as the solvent of the hydrogenated lecithin, and that the composition containing this solvent as well as the hyaluronic acid and the hydrogenated lecithin can improve the flexibility of the stratum corneum.

### <Test Example 2: Effect of Solvent 2>

In Test Example 2, a study was carried out on the effect of a trihydric alcohol (glycerin) as a solvent that dissolves the hydrogenated lecithin. The results are shown in Table 2 and Fig. 2.

### (Reference Example 3)

Ion-exchanged water was used as a test sample of Reference Example 3.

### (Reference Example 4: Control)

Ion-exchanged water was mixed with glycerin to prepare a test sample of Reference Example 4.

### (Comparative Example 1)

Hyaluronic acid was mixed with water to prepare an aqueous hyaluronic acid solution, and glycerin was mixed with hydrogenated lecithin to prepare a hydrogenated lecithin solution. Subsequently, under warming as appropriate, the hydrogenated lecithin solution was added to and mixed with the aqueous hyaluronic acid solution, to prepare a test sample of Comparative

### Example 1.

### [Table 2]

**Table 2**

| Component | Component name | Reference Example 3 | Reference Example 4 | Comparative Example 1 |
|---|---|---|---|---|
| Water | Ion-exchanged water | 100 | 90 | 89.72 |
| Trihydric alcohol | Glycerin | | 10 | 10 |
| Hyaluronic acid | Hyaluronic acid | | | 0.27 |
| Lecithin | Hydrogenated lecithin ¹⁾ | | | 0.01 |
| Total | | 100 | 100 | 100 |
| Mass ratio | | - | - | 27 |
| Evaluation | Flexibility of stratum corneum | C | - | C |

| | | | | |
|---|---|---|---|---|
| 1) Content of phosphatidylcholine: 75 to 85%; iodine number: 10 or less | | | | |

### <Results>

From the results in Table 2 and Fig. 2, it could be confirmed that the trihydric alcohol (glycerin) can be used as the solvent of the hydrogenated lecithin, but that the trihydric alcohol does not allow improvement of the flexibility of the stratum corneum by the inclusion of the hyaluronic acid and the hydrogenated lecithin in the composition, in contrast to the result of Test Example 1. These results showed that use of not only hyaluronic acid and hydrogenated lecithin, but also a particular solvent that dissolves the hydrogenated lecithin, that is, a dihydric alcohol, is important for improvement of the flexibility of the stratum corneum.

### <Test Example 3: Effects of Hyaluronic Acid, Hydrogenated Lecithin, and Dihydric Alcohol>

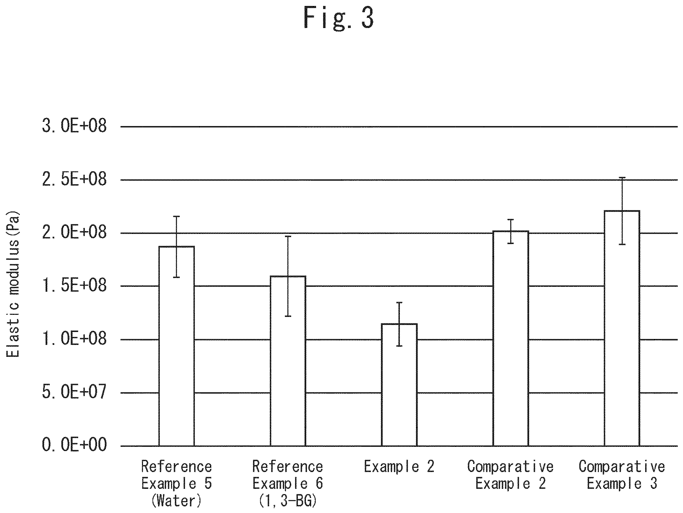

In Test Example 3, the effects of systems using hyaluronic acid alone or hydrogenated lecithin alone, and a mixed system using the three components, that is, hyaluronic acid, hydrogenated lecithin, and a dihydric alcohol, on improvement of the flexibility of the stratum corneum were studied. The results are shown in Table 3 and Fig. 3.

### (Reference Example 5)

Ion-exchanged water was used as a test sample of Reference Example 5.

### (Reference Example 6: Control)

Ion-exchanged water was mixed with 1,3-butylene glycol to prepare a test sample of Reference Example 6.

### (Example 2)

A test sample of Examples 2 was prepared in the same manner as in Example 1.

### (Comparative Example 2)

Hyaluronic acid was mixed with water to prepare an aqueous hyaluronic acid solution. Subsequently, under warming as appropriate, 1,3-butylene glycol was added to and mixed with the aqueous hyaluronic acid solution, to prepare a test sample of Comparative Example 2.

### (Comparative Example 3)

1,3-Butylene glycol was mixed with hydrogenated lecithin to prepare a hydrogenated lecithin solution. Subsequently, under warming as appropriate, the hydrogenated lecithin solution was added to and mixed with ion-exchanged water, to prepare a test sample of Comparative Example 3.

### [Table 3]

**Table 3**

| Component | Component name | Reference Example 5 | Reference Example 6 | Example 2 | Comparative Example 2 | Comparative Example 3 |
|---|---|---|---|---|---|---|
| Water | Ion-exchanged water | 100 | 90 | 89.72 | 89.73 | 89.99 |
| Dihydric alcohol | 1,3-Butylene glycol | | 10 | 10 | 10 | 10 |
| Hyaluronic acid | Hyaluronic acid | | | 0.27 | 0.27 | |
| Lecithin | Hydrogenated lecithin¹⁾ | | | 0.01 | | 0.01 |
| Total | | 100 | 100 | 100 | 100 | 100 |
| Mass ratio | | - | - | 27 | - | - |
| Evaluation | Flexibility of stratum corneum | C | - | A | C | C |

| | | | | | | |
|---|---|---|---|---|---|---|
| 1) Content of phosphatidylcholine: 75 to 85%; iodine number: 10 or less | | | | | | |

### <Results>

From the results in Table 3 and Fig. 3, it could be confirmed that, while use of hyaluronic acid alone or hydrogenated lecithin alone is not effective for improvement of the flexibility of the stratum corneum, use of the mixed system of the three components, that is, hyaluronic acid, hydrogenated lecithin, and a dihydric alcohol, is effective.

### <Test Example 4: Effect of Mass Ratio of Hyaluronic Acid to Hydrogenated Lecithin 1>

In Test Example 4, the amount of hydrogenated lecithin included was adjusted to study the effect of the mass ratio of hyaluronic acid to hydrogenated lecithin. The results are shown in Table 4 and Fig. 4.

### (Reference Example 7)

Ion-exchanged water was used as a test sample of Reference Example 7.

### (Reference Example 8: Control)

Ion-exchanged water was mixed with 1,3-butylene glycol to prepare a test sample of Reference Example 8.

### (Examples 3 and 4, and Comparative Examples 4 to 8)

According to the formulations in Table 4, test samples of Examples 3 and 4, and Comparative Examples 4 to 8 were prepared in the same manner as in Example 1.

### [Table 4]

**Table 4**

| Component | Component name | Reference Example 7 | Reference Example 8 | Comparative Example 4 | Comparative Example 5 | Comparative Example 6 | Example 3 | Example 4 | Comparative Example 7 | Comparative Example 8 |
|---|---|---|---|---|---|---|---|---|---|---|
| Water | Ion-exchanged water | 100 | 90 | 89.63 | 89.68 | 89.70 | 89.72 | 89.73 | 89.73 | 89.73 |
| Dihydric alcohol | 1,3-Butylene glycol | | 10 | 10 | 10 | 10 | 10 | 10 | 10 | 10 |
| Hyaluronic acid | Hyaluronic acid | | | 0.27 | 0.27 | 0.27 | 0.27 | 0.27 | 0.27 | 0.27 |
| Lecithin | Hydrogenated lecithin¹⁾ | | | 0.1 | 0.05 | 0.03 | 0.01 | 0.005 | 0.003 | 0.001 |
| Total | | 100 | 100 | 100 | 100 | 100 | 100 | 100 | 100 | 100 |
| Mass ratio | | - | - | 2.7 | 5.4 | 9.0 | 27 | 54 | 90 | 270 |
| Evaluation | Flexibility of stratum corneum | C | - | B | B | B | A | A | B | B |

| | | | | | | | | | | |
|---|---|---|---|---|---|---|---|---|---|---|
| 1) Content of phosphatidylcholine: 75 to 85%; iodine number: 10 or less | | | | | | | | | | |

### <Results>

From the results in Table 4 and Fig. 4, it could be confirmed that the improvement of the flexibility of the stratum corneum can be more effectively achieved in cases where the mass ratio of the hyaluronic acid to the hydrogenated lecithin is more than 9.0 and less than 90.

### <Test Example 5: Effect of Mass Ratio of Hyaluronic Acid to Hydrogenated Lecithin 2>

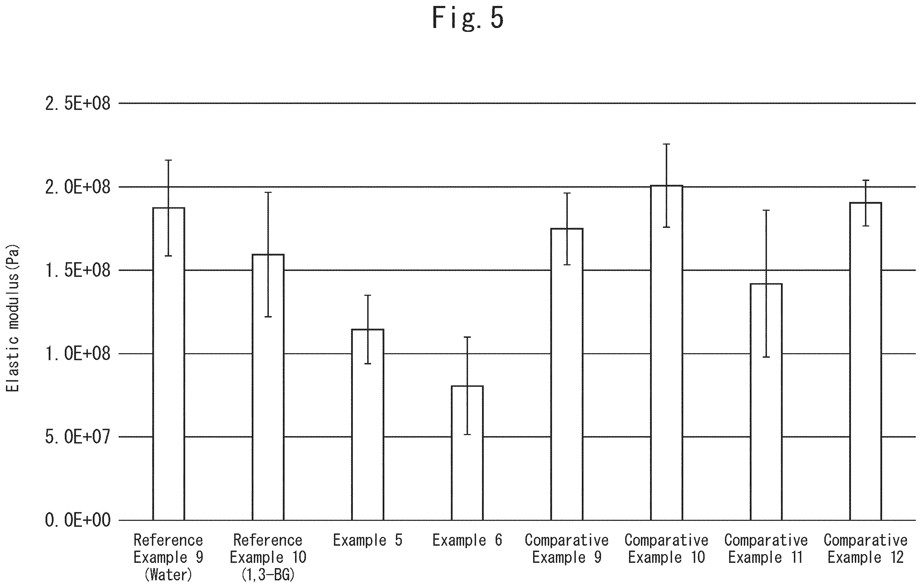

In Test Example 5, the amount of hyaluronic acid included was adjusted to study the effect of the mass ratio of hyaluronic acid to hydrogenated lecithin. The results are shown in Table 5 and Fig. 5. Regarding Example 6, the amount of hydrogenated lecithin included was set to 0.005% by mass in order to allow simple comparison with the above-described Example 4.

### (Reference Example 9)

Ion-exchanged water was used as a test sample of Reference Example 9.

### (Reference Example 10: Control)

Ion-exchanged water was mixed with 1,3-butylene glycol to prepare a test sample of Reference Example 10.

### (Examples 5 and 6, and Comparative Examples 9 to 12)

According to the formulations in Table 5, test samples of Examples 5 and 6, and Comparative Examples 9 to 12 were prepared in the same manner as in Example 1.

### [Table 5]

**Table 5**

| Component | Component name | Reference Example 9 | Reference Example 10 | Example 5 | Example 6 | Comparative Example 9 | Comparative Example 10 | Comparative Example 11 | Comparative Example 12 |
|---|---|---|---|---|---|---|---|---|---|
| Water | Ion-exchanged water | 100 | 90 | 89.72 | 89.91 | 89.90 | 89.95 | 89.96 | 89.98 |
| Dihydric alcohol | 1,3-Butylene glycol | | 10 | 10 | 10 | 10 | 10 | 10 | 10 |
| Hyaluronic acid | Hyaluronic acid | | | 0.27 | 0.09 | 0.09 | 0.045 | 0.027 | 0.009 |
| Lecithin | Hydrogenated lecithin¹⁾ | | | 0.01 | 0.005 | 0.01 | 0.01 | 0.01 | 0.01 |
| Total | | 100 | 100 | 100 | 100 | 100 | 100 | 100 | 100 |
| Mass ratio | | - | - | 27 | 18 | 9.0 | 4.5 | 2.7 | 0.9 |
| Evaluation | Flexibility of stratum corneum | C | - | A | A | C | C | B | C |

| | | | | | | | | | |
|---|---|---|---|---|---|---|---|---|---|
| 1) Content of phosphatidylcholine: 75 to 85%; iodine number: 10 or less | | | | | | | | | |

### <Results>

From the results in Table 5 and Fig. 5, it could be confirmed, similarly to Test Example 4, that the improvement of the flexibility of the stratum corneum can be more effectively achieved in cases where the mass ratio of the hyaluronic acid to the hydrogenated lecithin is more than 9.0 and less than 90.

### <Test Example 6: Skin Penetrability of Hyaluronic Acid 1>

In Test Example 6, a study was carried out on the skin penetrability of hyaluronic acid for a case where a composition having a mass ratio of hyaluronic acid to hydrogenated lecithin within the prescribed range was applied to the skin. The results are shown in Table 6 and Figs. 6 and 7.

### (Comparative Example 13: Control)

Hyaluronic acid was mixed with water to prepare an aqueous hyaluronic acid solution. Subsequently, under warming as appropriate, 1,3-butylene glycol was added to and mixed with the aqueous hyaluronic acid solution, to prepare a test sample of Comparative Example 13.

### (Example 7)

A test sample of Example 7 was prepared in the same manner as in Example 1.

### [Table 6]

**Table 6**

| Component | Component name | Comparative Example 13 | Example 7 |
|---|---|---|---|
| Water | Ion-exchanged water | 89.70 | 89.69 |
| Dihydric alcohol | 1,3-Butylene glycol | 10 | 10 |
| Hyaluronic acid | Hyaluronic acid | 0.30 | 0.30 |
| Lecithin | Hydrogenated lecithin¹⁾ | | 0.01 |
| Total | | 100 | 100 |
| Mass ratio | | - | 30 |
| Evaluation | Penetrability of HA | - | Good |

| | | | |
|---|---|---|---|
| 1) Content of phosphatidylcholine: 75 to 85%; iodine number: 10 or less | | | |

### <Results>

From the results in Table 6 and Figs. 6 and 7, it could be confirmed that a composition comprising hydrogenated lecithin, hyaluronic acid, and a dihydric alcohol wherein the mass ratio of the hyaluronic acid to the hydrogenated lecithin is within the prescribed range allows penetration of the hyaluronic acid into the stratum corneum. These results indicate that the flexibility-improving effect on the stratum corneum described above may be due to the fact that the moisture content in the stratum corneum was increased by the penetration of the hyaluronic acid into the stratum corneum.

### <Test Example 7: Skin Penetrability of Hyaluronic Acid 2>

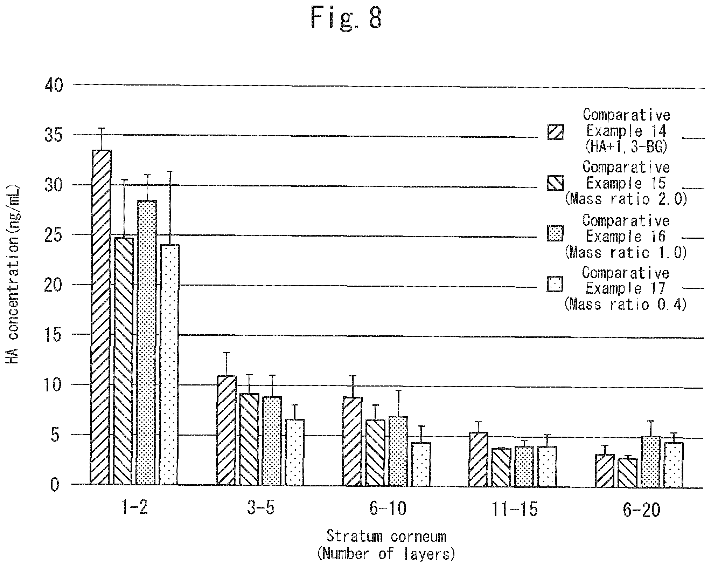

In Test Example 7, a study was carried out on the skin penetrability of hyaluronic acid for cases where a composition having a mass ratio of hyaluronic acid to hydrogenated lecithin outside the prescribed range was applied to the skin. The results are shown in Table 7, and Figs. 8 and 9.

### (Comparative Example 14: Control)

Hyaluronic acid was mixed with water to prepare an aqueous hyaluronic acid solution. Subsequently, under warming as appropriate, 1,3-butylene glycol was added to and mixed with the aqueous hyaluronic acid solution, to prepare a test sample of Comparative Example 14.

### (Comparative Example 15 to 17)

According to the formulations in Table 7, test samples of Comparative Examples 15 to 17 were prepared in the same manner as in Example 1.

### [Table 7]

**Table 7**

| Component | Component name | Comparative Example 14 | Comparative Example 15 | Comparative Example 16 | Comparative Example 17 |
|---|---|---|---|---|---|
| Water | Ion-exchanged water | 89.70 | 89.55 | 89.40 | 89.00 |
| Dihydric alcohol | 1,3-Butylene glycol | 10 | 10 | 10 | 10 |
| Hyaluronic acid | Hyaluronic acid | 0.30 | 0.30 | 0.30 | 0.30 |
| Lecithin | Hydrogenated lecithin¹⁾ | | 0.15 | 0.30 | 0.70 |
| Total | | 100 | 100 | 100 | 100 |
| Mass ratio | | - | 2.0 | 1.0 | 0.4 |
| Evaluation | Penetrability of HA | - | Poor | Poor | Poor |

| | | | | | |
|---|---|---|---|---|---|
| 1) Content of phosphatidylcholine: 75 to 85%; iodine number: 10 or less | | | | | |

### <Results>

From the results in Table 7 and Figs. 8 and 9, it could be confirmed that, even in cases where hydrogenated lecithin, hyaluronic acid, and a dihydric alcohol are contained, penetration of the hyaluronic acid into the stratum corneum is less likely to occur when the mass ratio of the hyaluronic acid to the hydrogenated lecithin is outside the prescribed range.

## Claims

1. A composition comprising:
hydrogenated lecithin;
hyaluronic acid; and
a dihydric alcohol,
wherein the mass ratio of the hyaluronic acid to the hydrogenated lecithin is more than 9.0 and less than 90.

2. The composition according to claim 1, wherein the content of phosphatidylcholine among constituent lipids of the hydrogenated lecithin is 40% or more.

3. The composition according to claim 1 or 2, wherein the content of the hyaluronic acid is 1.0% by mass or less with respect to the total amount of the composition.

4. The composition according to claim 1 or 2, wherein the content of the hydrogenated lecithin is 0.001% by mass or more with respect to the total amount of the composition.

5. The composition according to claim 1 or 2, wherein the content of the dihydric alcohol is 0.01% by mass or more with respect to the total amount of the composition.

6. The composition according to claim 1 or 2, wherein the dihydric alcohol is at least one selected from the group consisting of dipropylene glycol and 1,3-butylene glycol.

7. The composition according to claim 1 or 2, for use in penetration of the hyaluronic acid into body surface or body hair.

8. A cosmetic comprising the composition according to claim 1 or 2.
